# EUROPEAN PATENT APPLICATION

(11) **EP 4 406 517 A1**
(43) Date of publication of application: **31.07.2024**
(21) Application number: 21958354.9
(22) Date of filing: 22.09.2021
(51) Int. Cl.: A61F 2/90

(54) **STENT AND STENT DELIVERY SYSTEM**

(71) Applicant: Olympus Corporation, Hachioji-shi Tokyo 192-8507 (JP)
(72) Inventor: SASAKI, Kanta, Hachioji-shi, Tokyo 192-8507 (JP); NOGUCHI, Shun, Hachioji-shi, Tokyo 192-8507 (JP)
(74) Representative: Schicker, Silvia
(86) International application number: PCT/JP2021/034669
(87) International publication number: WO 2023/047477

(57) **Abstract**

Disclosed is a stent formed by weaving a wire, including: a plurality of straight line intersection portions at which straight line portions intersect with each other; and a plurality of engagement portions at which a mountain-shaped bent portion that is bent toward a side in a first direction, which is one side in an axial direction, and becomes convex, and a valley-shaped bent portion that is bent toward a side in a second direction, which is the other side in the axial direction, and becomes convex intersect with each other, wherein the plurality of straight line intersection portions have a first straight line intersection portion and a second straight line intersection portion, wherein the plurality of engagement portions have a first engagement portion, a second engagement portion, a third engagement portion, and a fifth engagement portion, wherein the first engagement portion and the second engagement portion are disposed at different positions in the axial direction, and wherein the second engagement portion and the third engagement portion are disposed at different positions in the axial direction.

## Description

### [Technical Field]

The present invention relates to a stent and a stent delivery system.

### [Background Art]

A technique for placing a stent in a stenosis or occlusion (hereinafter referred to as a "stenosis or the like") that occurs in the gastrointestinal tract or the like and expanding it is known. A stent delivery system is used to place a stent in the stenosis or the like. The stent delivery system is inserted into a treatment instrument channel of an endoscope to deliver the stent to the stenosis or the like.

For example, a stent described in Patent Document 1 is formed by hooking two bending points bent in a V shape on each other, and has high shape followability even when curved.

### [Citation List]

### [Patent Document]

[Patent Document 1]
Japanese Unexamined Patent Application, First Publication No. H7-265438

### [Summary of Invention]

### [Technical Problem]

However, when a stent including a portion with high shape followability, such as a form in which the bending points are hooked on each other as described in Patent Document 1, is accommodated in a stent delivery system, the portion with high shape followability in which the wires overlap each other multiple times is easily bulked up and difficult to be accommodated therein. Since a part in which the portion with high shape followability is bulked up has a strong diameter expansion force, the accommodated stent has a large sliding resistance between the stent and the stent delivery system, and thus it is difficult to release the stent.

In view of the above circumstances, an object of the present invention is to provide a stent that has high shape followability and is easily accommodated and released, and a stent delivery system equipped with the stent.

### [Solution to Problem]

In order to solve the above problems, the present invention proposes the following means.

According to a first aspect of the present invention, there is provided a stent formed by weaving a wire, including: a plurality of straight line intersection portions at which straight line portions intersect with each other; and a plurality of engagement portions at which a mountain-shaped bent portion that is bent toward a side in a first direction, which is one side in an axial direction, and becomes convex, and a valley-shaped bent portion that is bent toward a side in a second direction, which is the other side in the axial direction, and becomes convex intersect with each other, wherein the plurality of straight line intersection portions have a first straight line intersection portion at which a first straight line portion and a second straight line portion intersect with each other, and a second straight line intersection portion at which a third straight line portion and a fourth straight line portion intersect with each other, wherein the plurality of engagement portions have a first engagement portion at which a first mountain that is the mountain-shaped bent portion continuous to a side in the first direction of the first straight line portion and a first valley that is the valley-shaped bent portion intersect with each other, a second engagement portion at which a second mountain that is the mountain-shaped bent portion continuous to a side in the first direction of the second straight line portion and the fourth straight line portion and a second valley that is the valley-shaped bent portion intersect with each other, a third engagement portion at which a third mountain that is the mountain-shaped bent portion continuous to a side in the first direction of the third straight line portion and a third valley that is the valley-shaped bent portion intersect with each other, and a fifth engagement portion at which a fifth valley that is the valley-shaped bent portion continuous to a side in the second direction of the first straight line portion and the third straight line portion and a fifth mountain that is the mountain-shaped bent portion intersect with each other, wherein the first engagement portion and the second engagement portion are disposed at different positions in the axial direction, and wherein the second engagement portion and the third engagement portion are disposed at different positions in the axial direction.

According to a second aspect of the present invention, there is provided a stent delivery system including: an operation part; an outer tube member configured to extend to a distal side from the operation part; an inner tube member configured to extend to a distal side from the operation part and located on an inside of the outer tube member; and the above-described stent which is accommodated between the outer tube member and the inner tube member, wherein the operation part is configured to place the stent by moving the outer tube member or the inner tube member in a longitudinal direction.

### [Advantageous Effects of Invention]

The stent of the present invention has high shape followability and is easily accommodated and released.

### [Brief Description of Drawings]

FIG. 1 is a diagram showing the overall configuration of an endoscope system including a stent according to a first embodiment of the present invention.
FIG. 2 is a diagram showing the overall configuration of the same stent.
FIG. 3 is a development view of the same stent developed in a circumferential direction.
FIG. 4 is an enlarged view of a region F1 shown in FIGS. 2 and 3.
FIG. 5 is a diagram illustrating a deviation and a pitch of the same stent.
FIG. 6 is a cross-sectional view of the distal end portion of a stent delivery system.
FIG. 7 is a diagram showing the same stent with a large deviation.
FIG. 8 is a development view of a modification example of the same stent.
FIG. 9 is a development view of a stent according to a second embodiment of the present invention developed in the circumferential direction.
FIG. 10 is an enlarged view of a region F2 shown in FIG. 9.
FIG. 11 shows the results of measuring the expansion force of the stent according to the first embodiment.

### [Description of Embodiments]

### (First embodiment)

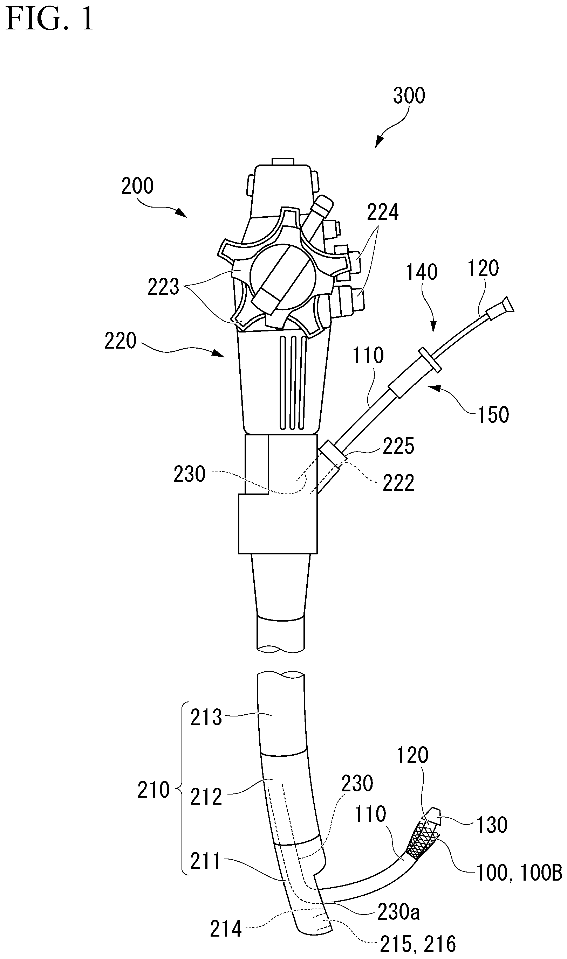

An endoscope system 300 equipped with a stent 100 according to a first embodiment of the present invention will be described with reference to FIGS. 1 to 7. FIG. 1 is a diagram showing the overall configuration of the endoscope system 300.

### [Endoscope system 300]

The endoscope system 300 includes an endoscope 200 and a stent delivery system 150 inserted into a channel of the endoscope 200.

### [Endoscope 200]

The endoscope 200 is a known side-viewing flexible endoscope and includes a long insertion section 210 and an operation section 220 provided at the proximal end portion of the insertion section 210. The endoscope 200 may be a direct viewing flexible endoscope.

The insertion section 210 includes a distal end rigid portion 211 provided at the distal end portion thereof, a curving portion 212 which is provided on the proximal end side of the distal end rigid portion 211 and which can be curved, and a flexible tube portion 213 provided on the proximal end side of the curving portion 212. A light guide 215 and an imaging unit 216 having a CCD are provided on a side surface of the distal end rigid portion 211 and exposed to the outside.

A treatment instrument channel 230 through which a treatment instrument for an endoscope such as the stent delivery system 150 is inserted is formed in the insertion section 210. A distal end portion 230a of the treatment instrument channel 230 opens on the side surface of the distal end rigid portion 211. A proximal end portion of the treatment instrument channel 230 extends to the operation section 220.

The distal end rigid portion 211 of the treatment instrument channel 230 is provided with a rising base 214. A proximal end portion of the rising base 214 is rotatably supported by the distal end rigid portion 211. A rising base operation wire (not shown) fixed to a distal end portion of the rising base 214 extends to the proximal end side through the insertion section 210.

The curving portion 212 is configured to be able to be freely curved in an updown direction and a left-right direction. A distal end of the operation wire is fixed to the distal end side of the curving portion 212. The operation wire extends through the insertion section 210 to the operation section 220.

The proximal end side of the operation section 220 is provided with a knob 223 for operating the operation wire and a switch 224 for operating the imaging unit 216 and the like. A user can curve the curving portion 212 in a desired direction by operating the knob 223.

A forceps port 222 that communicates with the treatment instrument channel 230 is provided on the distal end side of the operation section 220. The user can insert a treatment instrument for an endoscope such as the stent delivery system 150 through the forceps port 222. A forceps plug 225 is attached to the forceps port 222 in order to prevent leakage of a bodily fluid.

### [Stent delivery system 150]

The stent delivery system 150 is formed to be elongated as a whole and includes a stent 100, an outer tube member 110, an inner tube member 120, and an operation part 140.

The outer tube member 110 is formed of a resin or the like, has a cylindrical shape, and has flexibility. The outer tube member 110 can be inserted into the treatment instrument channel 230 of the endoscope 200.

The inner tube member 120 has an outer diameter smaller than the inner diameter of the outer tube member 110 and can pass through the internal space (the lumen) of the outer tube member 110. The inner tube member 120 is formed of a resin or the like and has flexibility. A tip 130 having an outer diameter larger than the outer diameter of the outer tube member 110 is provided at the distal end of the inner tube member 120.

The stent 100 is accommodated in the distal end portion of the stent delivery system 150, as shown in FIG. 1. The stent 100 is accommodated in a gap between the inner tube member 120 and the outer tube member 110 in a state in which the inner tube member 120 is passed therethrough and the diameter thereof is reduced.

The operation part 140 is connected to the proximal end sides of the outer tube member 110 and the inner tube member 120, and is configured to be able to move the outer tube member 110 relative to the inner tube member 120 in a longitudinal direction. By operating the operation part 140, the operator moves the outer tube member 110 relative to the inner tube member 120 to expose the accommodated stent 100, and as a result, the stent 100 can be placed. Furthermore, when the stent 100 has been exposed, the operator can re-accommodate the stent 100 by operating the outer tube member 110 to move relative to the inner tube member 120 in an opposite direction.

### [Stent 100]

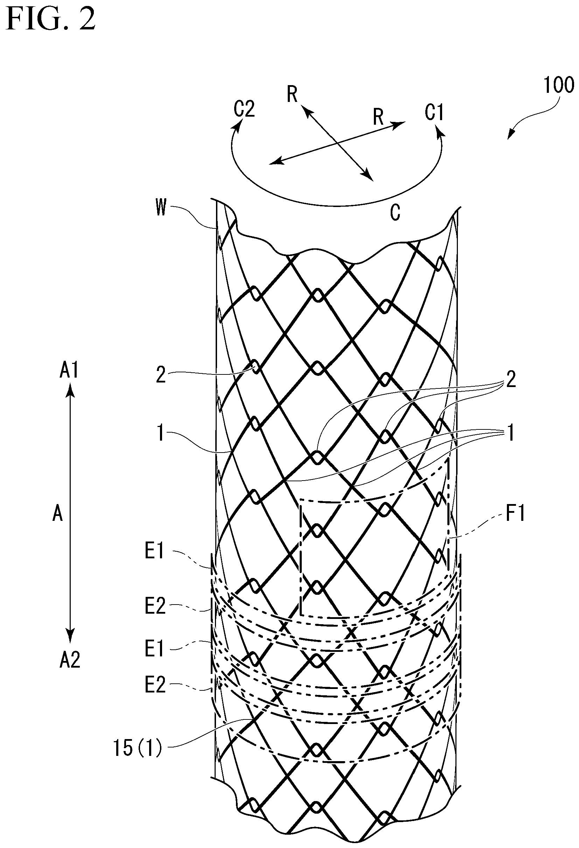

FIG. 2 is a diagram showing the overall configuration of the stent 100.

The stent 100 is formed by weaving a wire and has a cylindrical shape. The stent 100 is placed in a body lumen of a digestive system, such as a bile duct, esophagus, duodenum, small intestine, or large intestine, and is used primarily to expand and maintain the lumen.

The stent 100 of the present embodiment is not a so-called covered stent whose outer peripheral surface side is covered with a resin film or the like, but an uncovered stent which is not covered with a film or the like. However, the stent 100 can also be used as a covered stent by being covered with a resin film or the like.

In the following description, one side in a longitudinal axis direction (an axial direction) A of the stent 100 will be referred to as a "first direction A1," and the other side in the longitudinal axis direction A of the stent 100 will be referred to as a "second direction A2." Further, one side in a circumferential directions C of the stent 100 will be referred to as a "first circumferential direction C1," and the other side in the circumferential direction C of the stent 100 will be referred to as a "second circumferential direction C2."

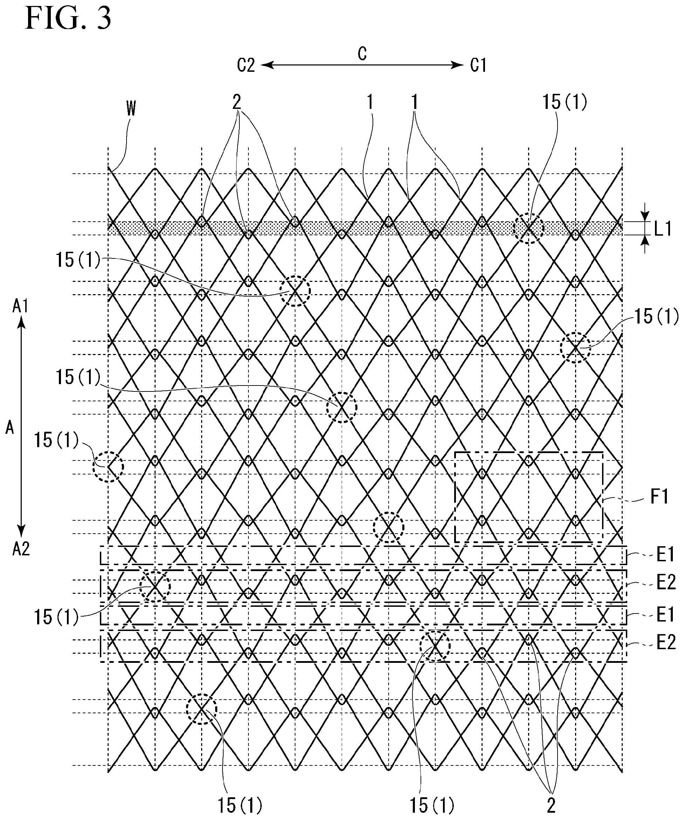

FIG. 3 is a development view of the stent 100 developed in the circumferential direction C.

The stent 100 is formed into a circular tube shape with a mesh on the circumferential surface by a wire W extending obliquely in a circumferential direction C while repeating bending. The stent 100 has a plurality of straight line intersection portions 1 and a plurality of engagement portions 2.

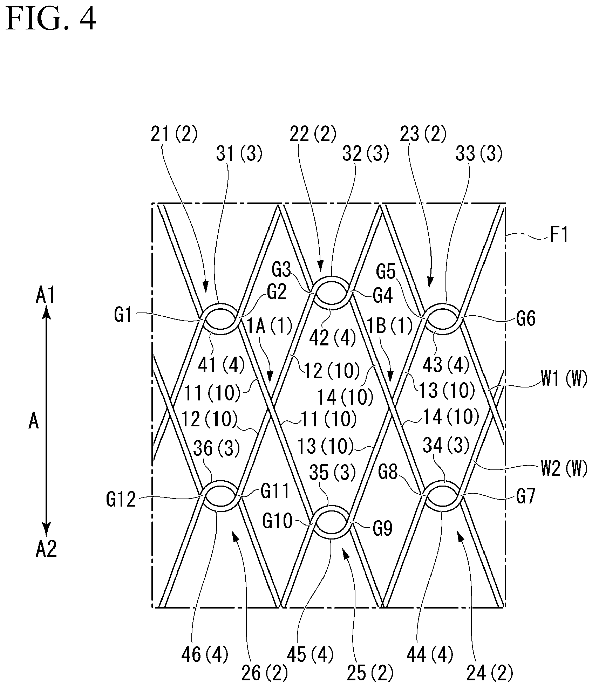

FIG. 4 is an enlarged view of a region F1 shown in FIGS. 2 and 3.

The straight line intersection portion 1 is formed by straight line portions 10 of the wires W intersecting with each other in a straight line. The straight line portion 10 is a portion of the wire W which extends obliquely in the circumferential direction C and has a substantially straight line shape, and also includes a gently curved portion.

The engagement portion (an intertwining portion) 2 is formed by a mountain-shaped bent portion 3 and a valley-shaped bent portion 4 intersecting with each other. The mountain-shaped bent portion (a mountain) 3 is a convex portion that is convex toward a side in the first direction A1, in which the wire W extending obliquely in the circumferential direction C is bent back in the longitudinal axis direction A. The valley-shaped bent portion (a valley) 4 is a convex portion that is convex toward a side in the second direction A2 (a concave portion that is concave toward a side in the first direction A1), in which the wire W extending obliquely in the circumferential direction C is bent back in the longitudinal axis direction A. At the engagement portion 2, the mountain-shaped bent portion 3 and the valley-shaped bent portion 4 intersect with each other in a hook shape, and thus the mountain-shaped bent portion 3 and the valley-shaped bent portion 4 are connected to each other such that they can be moved relative to each other, although they cannot be separated from each other.

As shown in FIGS. 2 and 3, a first region E1 and a second region E2 are alternately disposed in the longitudinal axis direction A. The first region E1 is a region in which the plurality of straight line intersection portions 1 are arranged in the circumferential direction C. Further, the second region E2 is a region in which the plurality of engagement portions 2 and one straight line intersection portion 1 are arranged in the circumferential direction C. The one straight line intersection portion 1 disposed in the second region E2 is provided for the wire W to move in the longitudinal axis direction A (step up and step down).

In the following description, the one straight line intersection portion 1 disposed in the second region E2 will be also referred to as a "step movement intersection portion 15." As shown in FIG. 3, one step movement intersection portion 15 is disposed in each second region E2. A plurality of step movement intersection portions 15 are disposed at different positions in the circumferential direction C.

Since the stent 100 is provided with a plurality of second regions E2 in which the plurality of engagement portions 2 are arranged in the circumferential direction C, the stent 100 has high shape followability even when curved.

### [First straight line intersection portion 1A]

As shown in FIG. 4, a first straight line portion 11 and a second straight line portion 12, which are the straight line portions 10, intersect with each other at a "first straight line intersection portion 1A" that is the straight line intersection portion 1. Specifically, when viewed in a radial direction R of the stent 100 (see FIG. 2), the first straight line portion 11 and the second straight line portion 12 intersect with each other at the first straight line intersection portion 1A. At the first straight line intersection portion 1A, the first straight line portion 11 passes outside the second straight line portion 12 in the radial direction R.

### [Second straight line intersection portion 1B]

As shown in FIG. 4, a third straight line portion 13 and a fourth straight line portion 14, which are the straight line portions 10, intersect with each other at a "second straight line intersection portion 1B" that is the straight line intersection portion 1. Specifically, when viewed in the radial direction R of the stent 100 (see FIG. 2), the third straight line portion 13 and the fourth straight line portion 14 intersect with each other at the second straight line intersection portion 1B. At the second straight line intersection portion 1B, the third straight line portion 13 passes inside the fourth straight line portion 14 in the radial direction R.

The first straight line intersection portion 1A and the second straight line intersection portion 1B are the straight line intersection portions 1 that are adjacent to each other in the circumferential direction C. The first straight line intersection portion 1A and the second straight line intersection portion 1B are disposed in the same first region E1.

### [First engagement portion 21]

A "first mountain 31" which is the mountain-shaped bent portion 3 is continuous to a side in the first direction A1 of the first straight line portion 11. The first mountain 31 intersects with a first valley 41 which is the valley-shaped bent portion 4 to form a "first engagement portion 21" which is the engagement portion 2.

Specifically, when viewed in the radial direction R of the stent 100, the first mountain 31 and the first valley 41 intersect with each other at a first intersection portion G1 and a second intersection portion G2. The second intersection portion G2 is closer to the first straight line intersection portion 1A than the first intersection portion G1. At the first intersection portion G1, the first mountain 31 passes outside the first valley 41 in the radial direction R. At the second intersection portion G2, the first mountain 31 passes inside the first valley 41 in the radial direction R.

### [Second engagement portion 22]

A "second mountain 32" which is the mountain-shaped bent portion 3 is continuous to a side in the first direction A1 of the second straight line portion 12 and the fourth straight line portion 14. The second mountain 32 intersects with a second valley 42 which is the valley-shaped bent portion 4 to form a "second engagement portion 22" which is the engagement portion 2.

Specifically, when viewed in the radial direction R of the stent 100, the second mountain 32 and the second valley 42 intersect with each other at a third intersection portion G3 and a fourth intersection portion G4. The third intersection portion G3 is closer to the first straight line intersection portion 1A than the fourth intersection portion G4. The fourth intersection portion G4 is closer to the second straight line intersection portion 1B than the third intersection portion G3. At the third intersection portion G3, the second mountain 32 passes outside the second valley 42 in the radial direction R. At the fourth intersection portion G4, the second mountain 32 passes inside the second valley 42 in the radial direction R.

### [Third engagement portion 23]

A "third mountain 33" which is the mountain-shaped bent portion 3 is continuous to a side in the first direction A1 of the third straight line portion 13. The third mountain 33 intersects with a third valley 43 which is the valley-shaped bent portion 4 to form a "third engagement portion 23" which is the engagement portion 2.

Specifically, when viewed in the radial direction R of the stent 100, the third mountain 33 and the third valley 43 intersect with each other at a fifth intersection portion G5 and a sixth intersection portion G6. The fifth intersection portion G5 is closer to the second straight line intersection portion 1B than the sixth intersection portion G6. At the fifth intersection portion G5, the third mountain 33 passes outside the third valley 43 in the radial direction R. At the sixth intersection portion G6, the third mountain 33 passes inside the third valley 43 in the radial direction R.

### [Fourth engagement portion 24]

A "fourth valley 44" which is the valley-shaped bent portion 4 is continuous to a side in the second direction A2 of the fourth straight line portion 14. The fourth valley 44 intersects with a fourth mountain 34 which is the mountain-shaped bent portion 3 to form a "fourth engagement portion 24" which is the engagement portion 2.

Specifically, when viewed in the radial direction R of the stent 100, the fourth mountain 34 and the fourth valley 44 intersect with each other at a seventh intersection portion G7 and an eighth intersection portion G8. The eighth intersection portion G8 is closer to the second straight line intersection portion 1B than the seventh intersection portion G7. At the seventh intersection portion G7, the fourth valley 44 passes outside the fourth mountain 34 in the radial direction R. At the eighth intersection portion G8, the fourth valley 44 passes inside the fourth mountain 34 in the radial direction R.

### [Fifth engagement portion 25]

A "fifth valley 45" which is the valley-shaped bent portion 4 is continuous to a side in the second direction A2 of the first straight line portion 11 and the third straight line portion 13. The fifth valley 45 intersects with a fifth mountain 35 which is the mountain-shaped bent portion 3 to form a "fifth engagement portion 25" which is the engagement portion 2.

Specifically, when viewed in the radial direction R of the stent 100, the fifth mountain 35 and the fifth valley 45 intersect with each other at a ninth intersection portion G9 and a tenth intersection portion G10. The ninth intersection portion G9 is closer to the second straight line intersection portion 1B than the tenth intersection portion G10. The tenth intersection portion G10 is closer to the first straight line intersection portion 1A than the ninth intersection portion G9. At the ninth intersection portion G9, the fifth valley 45 passes outside the fifth mountain 35 in the radial direction R. At the tenth intersection portion G10, the fifth valley 45 passes inside the fifth mountain 35 in the radial direction R.

### [Sixth engagement portion 26]

A "sixth valley 46" which is the valley-shaped bent portion 4 is continuous to a side in the second direction A2 of the second straight line portion 12. The sixth valley 46 intersects with a sixth mountain 36 which is the mountain-shaped bent portion 3 to form a "sixth engagement portion 26" which is the engagement portion 2.

Specifically, when viewed in the radial direction R of the stent 100, the sixth mountain 36 and the sixth valley 46 intersect with each other at an eleventh intersection portion G11 and a twelfth intersection portion G12. The eleventh intersection portion G11 is closer to the first straight line intersection portion 1A than the twelfth intersection portion G12. At the eleventh intersection portion G11, the sixth valley 46 passes outside the sixth mountain 36 in the radial direction R. At the twelfth intersection portion G12, the sixth valley 46 passes inside the sixth mountain 36 in the radial direction R.

### [Arrangement of first engagement portion 21, second engagement portion 22, and third engagement portion 23]

The first engagement portion 21 and the second engagement portion 22 are disposed at different positions in the longitudinal axis direction A. Specifically, the second engagement portion 22 is disposed closer to a side in the first direction A1 than the first engagement portion 21 in the longitudinal axis direction A.

The second engagement portion 22 and the third engagement portion 23 are disposed at different positions in the longitudinal axis direction A. Specifically, the third engagement portion 23 is disposed closer to a side in the second direction A2 than the second engagement portion 22 in the longitudinal axis direction A.

### [Arrangement of fourth engagement portion 24, the fifth engagement portion 25, and the sixth engagement portion 26]

The fourth engagement portion 24 and the fifth engagement portion 25 are disposed at different positions in the longitudinal axis direction A. Specifically, the fifth engagement portion 25 is disposed closer to a side in the second direction A2 than the fourth engagement portion 24 in the longitudinal axis direction A.

The fifth engagement portion 25 and the sixth engagement portion 26 are disposed at different positions in the longitudinal axis direction A. Specifically, the sixth engagement portion 26 is disposed closer to a side in the first direction A1 than the fifth engagement portion 25 in the longitudinal axis direction A.

### [Wire W]

The first mountain 31, the first straight line portion 11, the fifth valley 45, the third straight line portion 13, and the third mountain 33 are a continuous part (a first wire W1) of the wire W extending in a zigzag manner in the circumferential direction C. Further, the sixth valley 46, the second straight line portion 12, the second mountain 32, the fourth straight line portion 14, and the fourth valley 44 are a continuous part (a second wire W2) of the wire W extending in a zigzag manner in the circumferential direction C. The first wire W1 and the second wire W2 may be one continuous wire or may be different wires.

### [Other straight line intersection portions 1 and engagement portions 2]

The first straight line intersection portion 1A and the second straight line intersection portion 1B which are adjacent straight line intersection portions 1, and the six engagement portions 2 (the first engagement portion 21, the second engagement portion 22, the third engagement portion 23, the fourth engagement portion 24, the fifth engagement portion 25, and the sixth engagement portion 26) which are continuous to the first straight line intersection portion 1A and the second straight line intersection portion 1B have the above-mentioned configuration. As shown in FIG. 3, the engagement portions 2 continuous to other adjacent straight line intersection portions 1 in the stent 100 have the same configuration as the above-mentioned configuration, except for the portions where the step movement intersection portions 15 are provided.

### [Arrangement of engagement portion 2 in second region E2]

As shown in FIG. 3, the engagement portions 2 arranged in the circumferential direction C in the second region E2 are disposed at different positions in the longitudinal axis direction A from the adjacent engagement portions 2. Specifically, the engagement portions 2 arranged in the circumferential direction C in the second region E2 are alternately disposed one by one in the longitudinal axis direction A.

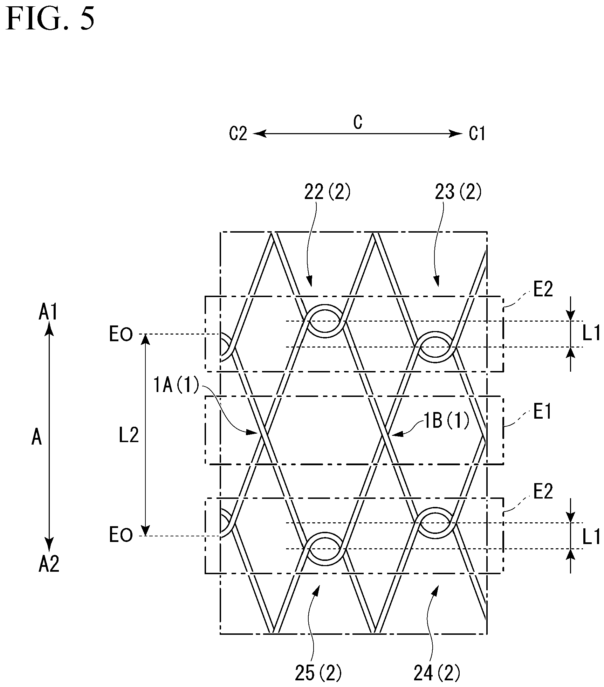

FIG. 5 is a diagram illustrating a deviation L1 and a pitch L2 of the same stent.

A length in the longitudinal axis direction A between a position of the engagement portion 2 that is closest to a side in the first direction A1 in the second region E2 and a position of the engagement portion 2 that is closest to a side in the second direction A2 in the same second region E2 is defined as a "deviation L1." Here, the position of the engagement portion 2 is a center position of the engagement portion 2 in the stent 100 that has naturally expanded into a straight line.

A length in the longitudinal axis direction A between the second regions E2 adjacent to each other in the longitudinal axis direction A is defined as a "pitch L2." Here, the length in the longitudinal axis direction A between the two second regions E2 is a distance between central axes EO of the second regions E2. The central axis EO is a straight line which passes through a midpoint in the longitudinal axis direction A between a position of the engagement portion 2 that is closest to a side in the first direction A1 in the second region E2 and a position of the engagement portion 2 that is closest to a side in the second direction A2 in the same second region E2, and is parallel to the circumferential direction C.

### [Operation of stent delivery system 150]

A stent placement method using the endoscope system 300 including the stent delivery system 150 will be described using a technique for placing the stent 100 in a bile duct as an example.

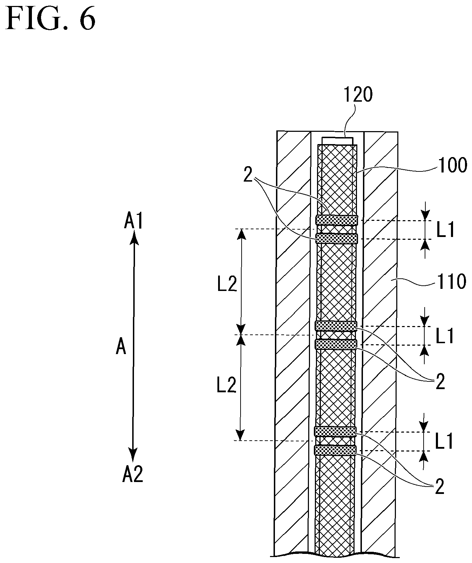

FIG. 6 is a cross-sectional view of the distal end portion of the stent delivery system 150.

The stent 100 is accommodated in a gap between the inner tube member 120 and the outer tube member 110 in a state in which the diameter thereof is reduced in the radial direction R. In the engagement portion 2 in which the wires W overlap each other multiple times, the wires W are easily bulked up in the radial direction R more than in other portions of the stent 100 in a state in which the diameter thereof is reduced in the radial direction R. However, in the stent 100, in the second region E2, the engagement portions 2 are disposed in a dispersed manner in the longitudinal axis direction A, and thus the bulk of the wires W in the radial direction R can be reduced. For this reason, the stent 100 is easily accommodated in the stent delivery system.

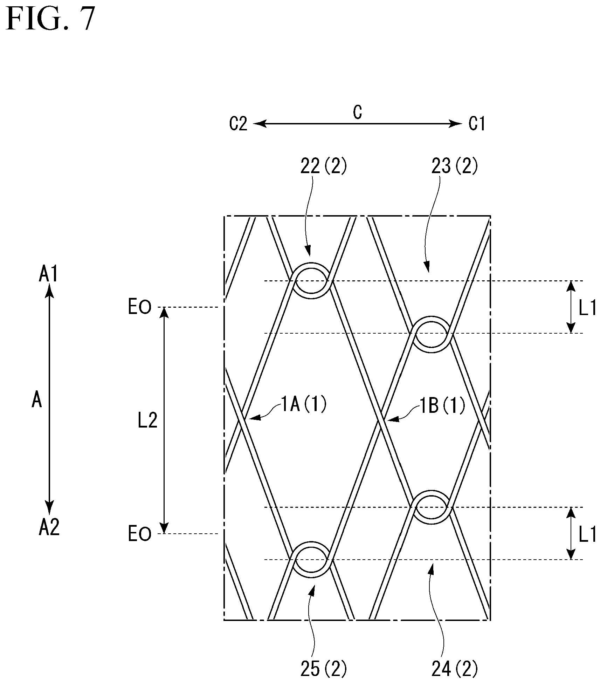

FIG. 7 is a diagram showing the stent 100 with a large deviation L1.

The larger the deviation L1 is, the more the stent 100 can suitably reduce the bulk of the wires W in the radial direction R in a state in which the diameter thereof is reduced in the radial direction R as shown in FIG. 6. Here, it is desirable that the deviation L1 be equal to or less than half the pitch L2. The bulk is most reduced when the deviation L1 is half the pitch L2. In a case where the deviation L1 is larger than half the pitch L2, the shape followability of the engagement portions 2 arranged in the circumferential direction C in the second region E2 will be reduced.

The operator inserts the insertion section 210 of the endoscope 200 into the patient's body cavity through a natural opening such as the mouth. At that time, the operator operates the knob 223 and the like to curve the curving portion 212 as necessary.

The operator passes a guide wire through the treatment instrument channel 230 of the endoscope 200 and inserts the guide wire into the bile duct while observing with the endoscope 200. Next, the operator operates the guide wire under X-ray fluoroscopy to break through the narrow portion in the bile duct, and moves the distal end portion of the guide wire to the liver side from the narrow portion (a target position).

The operator inserts the proximal end portion of the guide wire protruding from the forceps plug 225 of the endoscope 200 into a through hole of the tip 130 of the stent delivery system 150.

The operator advances the stent delivery system 150 along the guide wire by pushing the stent delivery system 150 while holding the guidewire. The distal end portion of the stent delivery system 150 protrudes from the distal end portion of the treatment instrument channel 230 of the endoscope 200. When the distal end portion of the stent delivery system 150 breaks through the narrow portion (the target position), the operator advances and retracts the stent delivery system 150 to determine the placement position of the stent 100. The operator may insert the stent delivery system 150 into the treatment instrument channel 230 without using the guide wire.

After the target position of the stent 100 is determined, the operator retracts the outer tube member 110 with respect to the inner tube member 120. As a result, as shown in FIG. 1, the stent 100 is gradually exposed and expanded from the distal end side.

In the state in which the diameter of the stent is reduced in the radial direction R, the portion in which the engagement portion 2 is bulked up and the wires W overlap each other multiple times has a strong diameter expansion force. However, in the stent 100, in the second region E2, the engagement portions 2 are disposed in a dispersed manner in the longitudinal axis direction A, and thus the diameter expansion force of the engagement portions 2 can be dispersed. For this reason, the accommodated stent has a small sliding resistance between the stent and the outer tube member 110 of the stent delivery system 150, and is therefore easily released.

When stent 100 is fully exposed, the stent 100 is expanded in its entirety such that the inner diameter of the stent 100 is greater than the outer diameter of inner tube member 120. Accordingly, the engagement between the stent 100 and the inner tube member 120 is released.

After the engagement between the stent 100 and the inner tube member 120 is released, when the operator retracts the inner tube member 120, the stent 100 remains in the placement position and the inner tube member 120 is removed from the stent 100.

When the operator pulls out the stent delivery system 150 except for the stent 100 from the body, the procedure for placing the stent 100 is completed.

According to the stent 100 of the present embodiment, the engagement portion 2 in which the wires W overlap each other multiple times is provided, and the shape followability is high. Furthermore, the engagement portions 2 in which the wires W overlap each other multiple times are disposed in a dispersed manner in the longitudinal axis direction A, and thus the stent 100 is easily accommodated in the stent delivery system 150 and easily released from the stent delivery system 150.

In the above, the first embodiment of the present invention has been described in detail with reference to the drawings, but the specific configuration is not limited to the embodiment, and a design change and the like within a range not departing from the gist of the present invention are also included. The constituent elements shown in the above-described embodiment and a modification example shown below can be appropriately combined and configured.

### (Modification Example 1-1)

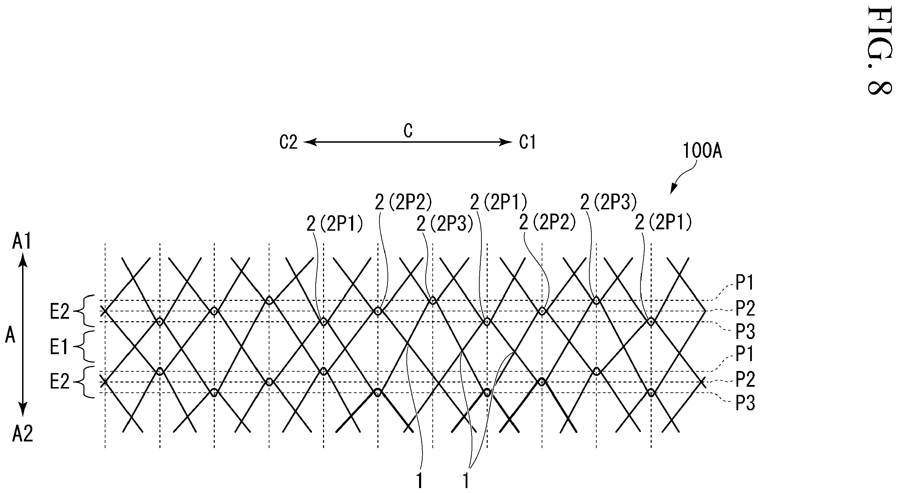

In the embodiment described above, the engagement portions 2 arranged in the circumferential direction C in the second region E2 are alternately disposed one by one in the longitudinal axis direction A. However, the arrangement aspect of the engagement portions 2 in the second region E2 is not limited to this. FIG. 8 is a development view of a stent 100A that is a modification example of the stent 100. The stent 100A differs from the stent 100 only in the arrangement aspect of the engagement portions 2 in the second region E2.

In the stent 100A, the engagement portions 2 arranged in the circumferential direction C in the second region E2 are disposed at one of a first position P1, a second position P2, and a third position P3 in the longitudinal axis direction A. The first position P1, the second position P2, and the third position P3 are arranged from a side in the first direction A1 toward a side in the second direction A2.

From a side in a first circumferential direction C1 toward a side in a second circumferential direction C2, the engagement portion 2 (2P1) disposed at the first position P1, the engagement portion 2 (2P2) disposed at the second position P2, and the engagement portion 2 (2P3) disposed at the third position P3 are repeatedly arranged. In the stent 100A, the engagement portions 2 in which the wires W overlap each other multiple times are disposed in a dispersed manner in the longitudinal axis direction A.

The plurality of engagement portions 2 arranged in the circumferential direction C in the second region E2 may be disposed in a dispersed manner at four or more different positions in the longitudinal axis direction A. In the engagement portions 2 adjacent to each other in the circumferential direction C in the second region E2, the engagement portion 2 on a side in the first circumferential direction C1 may be disposed on a side in the first direction A1 than a position of the engagement portion 2 on a side in the second circumferential direction C2 in the longitudinal axis direction A. Alternately, the engagement portion 2 on a side in the first circumferential direction C1 may be disposed on a side in the second direction A2 than a position of the engagement portion 2 on a side in the second circumferential direction C2 in the longitudinal axis direction A.

### (Second embodiment)

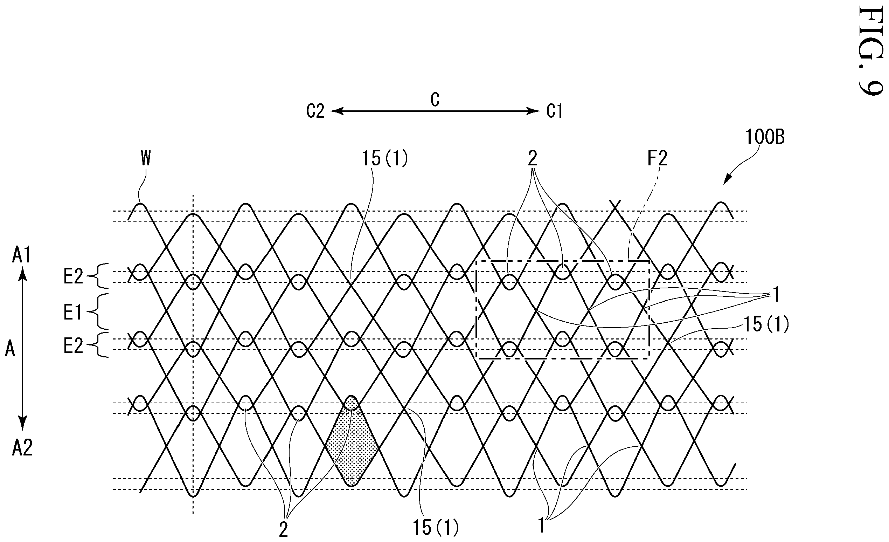

A second embodiment of the present invention will be described with reference to FIGS. 9 to 10. In the following description, the same constituent elements as those already described are designated by the same reference signs, and duplicate description will be omitted. A stent 100B according to the second embodiment is accommodated in a stent delivery system 150 similarly to the stent 100 according to the first embodiment.

FIG. 9 is a development view of the stent 100B developed in the circumferential direction C.

The stent 100B is formed by weaving a wire W and has a cylindrical shape similarly to the stent device 100 according to the first embodiment. Furthermore, the stent 100B has a plurality of straight line intersection portions 1 and a plurality of engagement portions 2. The stent 100B differs from the stent 100 of the first embodiment only in the arrangement of the engagement portions 2.

### [Arrangement of first engagement portion 21, second engagement portion 22, and third engagement portion 23]

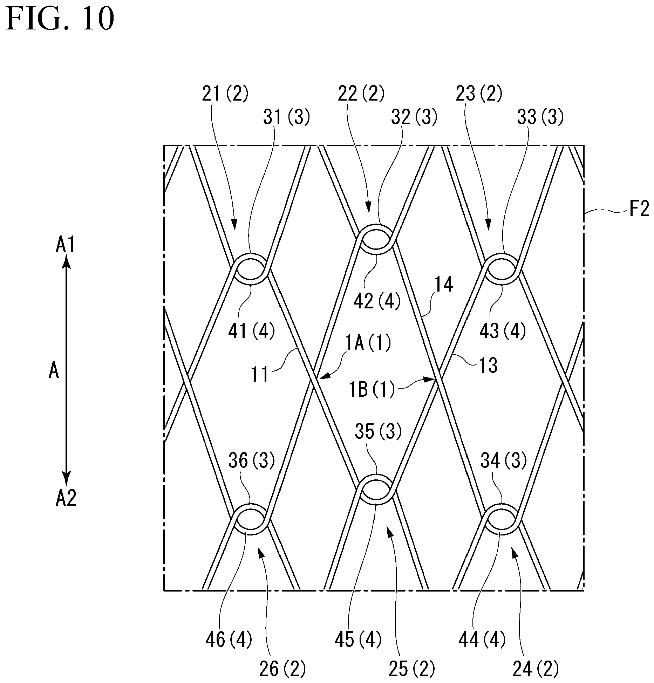

FIG. 10 is an enlarged view of a region F2 shown in FIG. 9.

The first engagement portion 21 and the second engagement portion 22 are disposed at different positions in the longitudinal axis direction A. Specifically, the second engagement portion 22 is disposed closer to a side in the first direction A1 than the first engagement portion 21 in the longitudinal axis direction A.

The second engagement portion 22 and the third engagement portion 23 are disposed at different positions in the longitudinal axis direction A. Specifically, the third engagement portion 23 is disposed closer to a side in the second direction A2 than the second engagement portion 22 in the longitudinal axis direction A.

### [Arrangement of fourth engagement portion 24, the fifth engagement portion 25, and the sixth engagement portion 26]

The fourth engagement portion 24 and the fifth engagement portion 25 are disposed at different positions in the longitudinal axis direction A. Specifically, the fifth engagement portion 25 is disposed closer to a side in the first direction A1 than the fourth engagement portion 24 in the longitudinal axis direction A.

The fifth engagement portion 25 and the sixth engagement portion 26 are disposed at different positions in the longitudinal axis direction A. Specifically, the sixth engagement portion 26 is disposed closer to a side in the second direction A2 than the fifth engagement portion 25 in the longitudinal axis direction A.

According to the stent 100B of the present embodiment, the engagement portion 2 in which the wires W overlap each other multiple times is provided, and the shape followability is high. Furthermore, the engagement portions 2 in which the wires W overlap each other multiple times are disposed in a dispersed manner in the longitudinal axis direction A, and thus the stent 100B is easily accommodated in the stent delivery system 150 and easily released from the stent delivery system 150.

In the above, the second embodiment of the present invention has been described in detail with reference to the drawings, but the specific configuration is not limited to the embodiment, and a design change and the like within a range not departing from the gist of the present invention are also included. The constituent elements shown in the above-described embodiment and a modification example can be appropriately combined and configured.

### [Examples]

Hereinafter, the present invention will be explained in detail on the basis of Examples, but the technical scope of the present invention is not limited to these Examples.

Example 1 is the stent 100 of the above embodiment.

Comparative Example 1 is a stent in which the deviation L 1 of the stent 100 is made zero.

Expansion force measurement was performed using Example 1 and Comparative Example 1. The expansion force measurement was carried out on the basis of "4. Measurement of radial force" specified in JIS T3269. Reduction and expansion of the diameter of the stent placed in a bath at 37 °C are alternately and repeatedly executed for the entire stent.

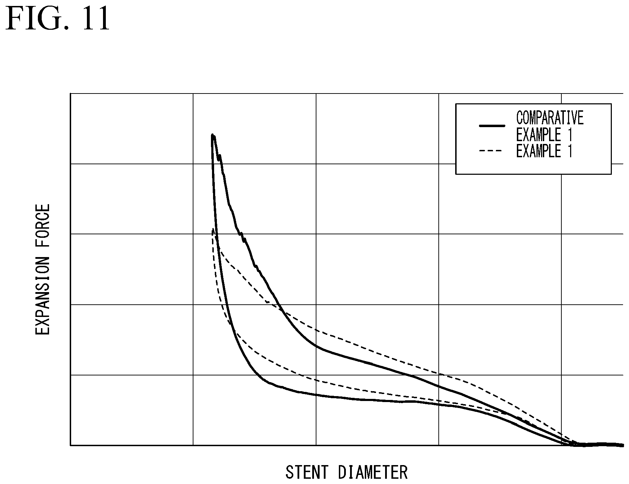

FIG. 11 shows the results of measuring the expansion force of the stent 100.

The expansion force of Example 1 is smaller than the expansion force of Comparative Example 1 in a state in which the diameter of the stent is most reduced. In Example 1, in the second region E2, the engagement portions 2 are disposed in a dispersed manner in the longitudinal axis direction A, and thus the bulk of the wires W in the radial direction R can be reduced and the diameter expansion force of the engagement portions 2 can be dispersed.

On the other hand, in the process of diameter reduction and diameter expansion, there is little difference between the expansion force of Example 1 and the expansion force of Comparative Example 1. That is, even in a case where the stent 100 is provided with the deviation L1 as in Example 1, the basic function of the stent, which is to widen the stenosis or occlusion, can be maintained.

### [Industrial Applicability]

The present invention can be applied to a stent formed by weaving a wire or the like.

### [Reference Signs List]

300 Endoscope system
200 Endoscope
150 Stent delivery system
110 Outer tube member
120 Inner tube member
130 Tip
100, 100A, 100B Stent
1 Straight line intersection portion
10 Straight line portion
11 First straight line portion
12 Second straight line portion
13 Third straight line portion
14 Fourth straight line portion
15 Step movement intersection portion
1A First straight line intersection portion
1B Second straight line intersection portion
2 Engagement portion (intertwining portion)
21 First engagement portion
22 Second engagement portion
23 Third engagement portion
24 Fourth engagement portion
25 Fifth engagement portion
26 Sixth engagement portion
3 Mountain-shaped bent portion (mountain)
31 First mountain
32 Second mountain
33 Third mountain
34 Fourth mountain
35 Fifth mountain
36 Sixth mountain
4 Valley-shaped bent portion (valley)
41 First Valley
42 Second valley
43 Third Valley
44 Fourth Valley
45 Fifth valley
46 Sixth Valley

## Claims

1. A stent formed by weaving a wire, comprising:
a plurality of straight line intersection portions at which straight line portions intersect with each other; and
a plurality of engagement portions at which a mountain-shaped bent portion that is bent toward a side in a first direction, which is one side in an axial direction, and becomes convex, and a valley-shaped bent portion that is bent toward a side in a second direction, which is the other side in the axial direction, and becomes convex intersect with each other,
wherein the plurality of straight line intersection portions have
a first straight line intersection portion at which a first straight line portion and a second straight line portion intersect with each other, and
a second straight line intersection portion at which a third straight line portion and a fourth straight line portion intersect with each other,
wherein the plurality of engagement portions have
a first engagement portion at which a first mountain that is the mountain-shaped bent portion continuous to a side in the first direction of the first straight line portion and a first valley that is the valley-shaped bent portion intersect with each other,
a second engagement portion at which a second mountain that is the mountain-shaped bent portion continuous to a side in the first direction of the second straight line portion and the fourth straight line portion and a second valley that is the valley-shaped bent portion intersect with each other,
a third engagement portion at which a third mountain that is the mountain-shaped bent portion continuous to a side in the first direction of the third straight line portion and a third valley that is the valley-shaped bent portion intersect with each other, and
a fifth engagement portion at which a fifth valley that is the valley-shaped bent portion continuous to a side in the second direction of the first straight line portion and the third straight line portion and a fifth mountain that is the mountain-shaped bent portion intersect with each other,
wherein the first engagement portion and the second engagement portion are disposed at different positions in the axial direction, and
wherein the second engagement portion and the third engagement portion are disposed at different positions in the axial direction.

2. The stent according to claim 1,
wherein the second engagement portion is disposed closer to a side in the first direction than the first engagement portion in the axial direction, and
wherein the third engagement portion is disposed closer to a side in the second direction than the second engagement portion in the axial direction.

3. The stent according to claim 1, wherein a first region in which the plurality of straight line intersection portions are disposed in a circumferential direction and a second region in which the plurality of engagement portions and one straight line intersection portion are disposed in the circumferential direction are disposed alternately in the axial direction.

4. The stent according to claim 3, wherein the straight line intersection portions disposed in the second regions are disposed at different positions in the circumferential direction.

5. The stent according to claim 3, wherein a length in the axial direction between a position of the engagement portion that is closest to a side in the first direction in the second region and a position of the engagement portion that is closest to a side in the second direction in the same second region is equal to or less than half a length in the axial direction between the second regions adjacent to each other in the axial direction.

6. The stent according to claim 1,
wherein the plurality of engagement portions further include
a fourth engagement portion at which a fourth valley that is the valley-shaped bent portion continuous to a side in the second direction of the fourth straight line portion and a fourth mountain that is the mountain-shaped bent portion intersect with each other, and
a sixth engagement portion at which a sixth valley that is the valley-shaped bent portion continuous to a side in the second direction of the second straight line portion and a sixth mountain that is the mountain-shaped bent portion intersect with each other,
wherein the fourth engagement portion and the fifth engagement portion are disposed at different positions in the axial direction, and
wherein the fifth engagement portion and the sixth engagement portion are disposed at different positions in the axial direction.

7. The stent according to claim 6,
wherein the second engagement portion is disposed closer to a side in the first direction than the first engagement portion in the axial direction,
wherein the third engagement portion is disposed closer to a side in the second direction than the second engagement portion in the axial direction,
wherein the fifth engagement portion is disposed closer to a side in the second direction than the fourth engagement portion in the axial direction, and
wherein the sixth engagement portion is disposed closer to a side in the first direction than the fifth engagement portion in the axial direction.

8. The stent according to claim 6,
wherein the second engagement portion is disposed closer to a side in the first direction than the first engagement portion in the axial direction,
wherein the third engagement portion is disposed closer to a side in the first direction than the second engagement portion in the axial direction,
wherein the fifth engagement portion is disposed closer to a side in the first direction than the fourth engagement portion in the axial direction, and
wherein the sixth engagement portion is disposed closer to a side in the first direction than the fifth engagement portion in the axial direction.

9. The stent according to claim 6,
wherein the second engagement portion is disposed closer to a side in the first direction than the first engagement portion in the axial direction,
wherein the third engagement portion is disposed closer to a side in the second direction than the second engagement portion in the axial direction,
wherein the fifth engagement portion is disposed closer to a side in the first direction than the fourth engagement portion in the axial direction, and
wherein the sixth engagement portion is disposed closer to a side in the second direction than the fifth engagement portion in the axial direction.

10. A stent delivery system comprising:
an operation part;
an outer tube member configured to extend to a distal side from the operation part;
an inner tube member configured to extend to a distal side from the operation part and located on an inside of the outer tube member; and
the stent according to any one of claims 1 to 9 which is accommodated between the outer tube member and the inner tube member,
wherein the operation part is configured to place the stent by moving the outer tube member or the inner tube member in a longitudinal direction.

11. The stent delivery system according to claim 10, wherein the outer tube member is configured to be able to be inserted into a channel of an endoscope.
